# EUROPEAN PATENT SPECIFICATION

(11) **EP 2 872 219 B1**
(45) Date of publication and mention of the grant of the patent: **06.09.2017**
(21) Application number: 13763117.2
(22) Date of filing: 03.07.2013
(51) Int. Cl.: A61Q 5/00, A61Q 9/04, A61K 8/11, A61K 8/14, A61K 8/46, A61K 8/86

(54) **RELEASE OF MOLECULES FROM CONTAINER BY SELECTIVE HEATING OF HAIR**
FREISETZUNG VON MOLEKÜLEN AUS EINEM BEHÄLTER DURCH SELEKTIVE ERWÄRMUNG VON HAAR
LIBÉRATION DE MOLÉCULES À PARTIR D'UN RÉCIPIENT PAR CHAUFFAGE SÉLECTIF DE CHEVEUX

(30) Priority: 10.07.2012 US 201261669699 P
(43) Date of publication of application: 20.05.2015
(73) Proprietor: Koninklijke Philips N.V., 5656 AE Eindhoven (NL)
(72) Inventor: MOESKOPS, Bastiaan Wilhelmus Maria, NL-5656 AE Eindhoven (NL); CIUHU, Calina, NL-5656 AE Eindhoven (NL); LANGEREIS, Sander, NL-5656 AE Eindhoven (NL); CHLON, Caecilia Hendrina Theodora, NL-5656 AE Eindhoven (NL)
(74) Representative: Coops, Peter
(86) International application number: PCT/IB2013/055447
(87) International publication number: WO 2014/009857

(56) References cited:
- EP-A1- 0 911 023
- WO-A1-94/22468
- WO-A1-97/38638
- WO-A1-2009/073017
- WO-A1-2011/019668
- FR-A1- 2 935 902
- GB-A- 2 396 556
- US-B1- 6 168 590

## Description

### FIELD OF THE INVENTION

Embodiments of the present invention relate generally to the field of hair treatments, and, more specifically, to methods for releasing chemical compounds onto hair.

### DESCRIPTION OF THE RELATED ART

Historically, a number of techniques have been used in eliminating unwanted hair on various body parts. One technique commonly utilized for the removal of hair on arms and legs relies on the use of depilatory creams containing bioactive ingredients, such as thioglycolates. Weakening of the hair occurs by the oxidation of the disulfide bridges of keratin, resulting in easy removal of the hair. One drawback of this technique is that, since the epidermis contains a high content of keratin, damaging and irritation of the skin occurs if the thioglycolate mixture is applied for an extended period of time. Another drawback is that thioglycolate-based creams, for instance Veet®, cannot be used in the face, due to the fact that facial hair dissolves too slowly using thioglycolates, giving rise to unacceptable damage of the skin.

Other techniques are based on thermolysis of hair achieved by irradiation of hair with light. For example, WO97/38638 and WO2009/073017 describe methods of permanent hair removal which rely on preferentially depositing additional quantities of melanin, a naturally occurring element in the skin and hair follicles, at the follicle sites or in their proximity. The preferential deposition is achieved by encapsulating melanin-based compounds in liposomes specifically selected and formed to bind to specific sites in the proximity of or inside the hair follicles. After a medium containing such liposomes has been applied topically to the skin, laser light of a frequency which is readily absorbed by the melanin is directed to the skin. As a consequence of the added quantity of melanin, a greater proportion of the incident light is absorbed at the hair follicles, causing thermolysis and death of the follicles. One drawback of this approach is that melanin-containing liposomes may also be deposited on areas which are not necessarily in the proximity of hair follicles, leading to undesirable increased absorption of light in those areas.

What is needed in the art is a method of providing chemical compounds for hair removal, hair coloring, or other treatment in a manner that reduces at least some of the drawbacks described above.

### SUMMARY OF THE INVENTION

According to one aspect of the invention, a method of releasing a chemical compound onto hair is disclosed. The method includes distributing containers encapsulating the chemical compound over hair, where the containers are configured to release the chemical compound upon reaching a transition temperature. The method also includes heating the hair to a predetermined temperature that is equal to or higher than the transition temperature. The heating of the hair enables heat transfer from the hair to one or more of the containers that are in proximity of the hair, whereby the one or more containers reach the transition temperature and release the chemical compound. In turn, the released chemical compound that is in contact with the hair may e.g. treat the hair.

The invention is based on the recognition that encapsulating a chemical compound into containers, distributing the containers over the hair, and selectively heating the hair in a manner that avoids or minimizes direct heating of the containers results in the hair being heated to a temperature higher than that of the containers and, therefore, enables heat transfer from the heated hair to the containers. When such containers are temperature-sensitive so that they are configured to release the compound upon reaching a certain transition temperature, and when the hair is heated to a temperature that is equal to or higher than the transition temperature of the containers, then the heat transfer from the heated hair to the containers will, eventually, heat the containers to their transition temperature and they will release the compound. In this manner, only those containers that are in close proximity to the hair, preferably adjacent to or in contact with the hair, can be heated up, via the heat transfer from the hair, to their transition temperature and are able to release the compound. Consequently, the compound is only released locally where the hair is present, while other skin areas where the hair is not present are protected against the potentially negative side effects of the compound.

In one embodiment, selective heating of hair may be achieved by irradiating the hair with light, the wavelength or the range of wavelengths of said light being selected so that a portion of the light absorbed by the hair is greater than a portion of the light absorbed by the containers and the chemical compound in order to ensure that the hair is heated up by the light to a higher temperature than the containers and that the containers only reach their transition temperature via the heat transfer from the hair. For safety reasons, the wavelengths of the light are preferably 400 nanometers (nm) or higher. In various embodiments, such light could be generated by one or more of a laser, a laser diode, a light emitting diode (LED) or an intense pulsed light (IPL) source such as a flash lamp.

In an alternative embodiment, selective heating of hair may be achieved using a pressure wave source, such as e.g. an ultrasound transducer.

In an embodiment, the temperature-sensitive containers could be in the form of carriers comprising a lipid bilayer shell. Particularly, such shells could enclose a cavity, and be semi-permeable, typically comprising phospholipids. The carriers could be microcarriers, having a particle size whose diameter is of the order of several microns to tens of microns, and nanocarriers, having a particle size of the order of tens to hundreds of nanometers. In the context of the invention, the carriers are hereinafter referred to as "liposomes". Liposomes are typically spherical vesicles comprising a bilayer membrane enclosing a cavity (referred to as a "lumen"). The bilayer can be made up of at least one phospholipid and may or may not comprise cholesterol. Temperature-sensitive liposomes (TSLs) release encapsulated molecules at the melting phase transition temperature (*T*ₘ) of the lipid bilayer.

In an embodiment, the containers could be distributed over the hair in the form of a topical formulation such as e.g. a cream, a spray, a gel, a suspension, or a solution carrying the containers.

In an embodiment, the step of distributing the containers over the hair could comprise distributing the containers over a skin area comprising the hair. Such containers would be selectively opened, releasing the chemical compound stored in them, only near the hair where sufficient heat is available but will remain closed at other locations. This enables easy application of the containers while protecting the skin against the chemicals.

In an embodiment, the chemical compound could be a compound for treatment of the hair that is in contact with the released compound, such as e.g. hair coloring, hair conditioning, hair removal, or any other treatment. For example, the chemical compound could include water-soluble components such as enzymes, hormones or other chemicals like e.g. thioglycolate.

A method according to the invention can be carried out by means of an assembly for releasing a chemical compound onto hair. The assembly includes a topical formulation for distribution over hair and a heat source. The topical formulation comprises containers encapsulating the chemical compound, the containers being configured to release the chemical compound upon reaching a transition temperature. The heat source is configured to selectively heat the hair to a predetermined temperature equal to or higher than the transition temperature such that, in use, a temperature of the hair increases faster than a temperature of the containers and the chemical compounds therein, whereby heat transfer from the hair to one or more of the containers that are in proximity of the hair is enabled, causing the one or more containers to reach the transition temperature and release the chemical compound. Such an assembly could be used in e.g. a light-based hair treatment product, such as a photoepilation device, or light-based shavers or groomers.

Hereinafter, embodiments of the invention will be described in further detail. It should be appreciated, however, that these embodiments may not be construed as limiting the scope of protection of the present invention.

### BRIEF DESCRIPTION OF THE DRAWINGS

In all figures, the dimensions as sketched are for illustration only and do not reflect the true dimensions or ratios. All figures are schematic and not to scale.
Fig. 1A illustrates a temperature-sensitive container below its transition temperature, encapsulating a chemical compound, for use in a method according to an embodiment of the present invention;
Fig. 1B illustrates the temperature-sensitive container of Fig. 1A at or above its transition temperature, releasing the chemical compound, for use in a method according to an embodiment of the present invention;
Fig. 2 illustrates removal of hair by temperature-induced release of bioactive ingredients from temperature-sensitive containers, according to an embodiment of the present invention;
Fig. 3 illustrates the release of hair dyes, conditioner or therapeutics, e.g. for hair coloring, from temperature-sensitive containers, according to an embodiment of the present invention;
Fig. 4 illustrates enhanced photoepilation, according to an embodiment of the present invention; and
Fig. 5 illustrates experimental proof of light-induced release of fluorescent chemical compound encapsulated in temperature-sensitive liposomes.

### DETAILED DESCRIPTION

In the following description, numerous specific details are set forth to provide a more thorough understanding of the present invention. However, it will be apparent to one of skill in the art that the present invention may be practiced without one or more of these specific details. In other instances, well-known features have not been described in order to avoid obscuring the present invention.

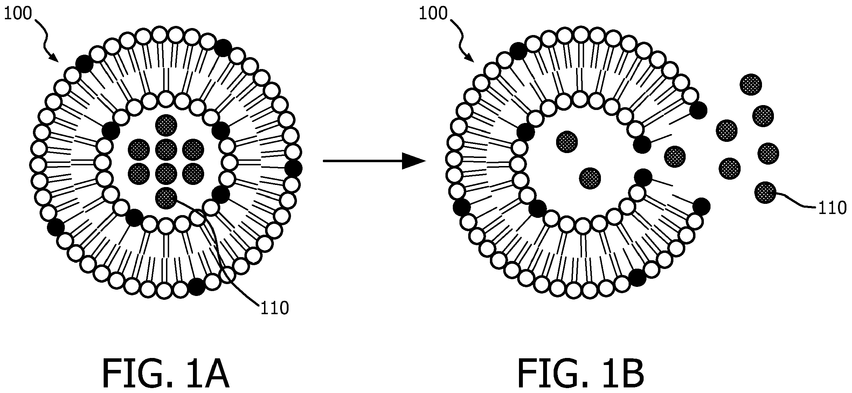

Figs. 1A and 1B each illustrate a temperature-sensitive container 100, for use in a method according to one embodiment of the present invention. When the container 100 is at a temperature below its transition temperature, the container 100 is closed and encapsulates a chemical compound 110, as shown in Fig. 1A. In this state, preferably, the chemical compound 110 does not escape from the container 100 at all, or, at least is not released from the container 100 in significant amounts. However, when the container 100 is heated to its phase transition temperature, the container 100 is configured so as to open and release the compound 110, as is shown in Fig. 1B. While Fig. 1B illustrates that the container 100 opens to release the compound 110 from one side (the right side in Fig. 1B), persons skilled in the art will recognize that, in other embodiments, the container 100 could release the compound 110 in other manners, all of these manners being within the scope of the present invention. For example, when the container 100 reaches its transition temperature, the walls of the container 100 could become porous, so that the compound 110 can sip out through the walls. Alternatively, upon reaching its transition temperature, the container 100 could partially or completely dissolve, thereby also releasing the compound 110.

The present invention provides a method of releasing the compound 110 from the container 100 onto hair in a manner that minimizes the amount of the compound 110 released onto anything other than the hair, e.g. the skin.

The method begins with distributing a plurality of containers 100 over hair. Upon distribution, the containers 100 are at a temperature that is below their transition temperature and, therefore, are closed. In various embodiments, the containers 100 could be distributed by being included in any kind of topical formulation such as e.g. a cream, a spray, a gel, a suspension, a solution, or a powder. For easy application, in an embodiment, the containers 100 could be distributed over the entire skin area comprising the hair, as opposed to being e.g. spread only onto hair and not onto the skin area near and/or under the hair.

Once the containers 100 are distributed over the hair, the hair is selectively heated. As used herein, the expression "selective heating" describes heating of the hair in such a manner that the temperature of the hair increases faster than the temperature of the containers 100 and the chemical compounds 110 therein.

In one embodiment, selective heating of hair may be achieved by irradiating the hair with light, the wavelength or the range of wavelengths of the light being selected so that a portion of the light absorbed by the hair is greater than a portion of the light absorbed by the containers 100 and the chemical compound 110. In a preferred embodiment, the containers 100 and the chemical compounds 110 do not absorb any light or absorb only a small amount, e.g. less than 10%, of the light incident on the containers and chemical compounds, while the hair readily absorbs the light, e.g. more than 50% of the light incident on the hair. The absorption behavior of the containers 100 and the compounds 110 could be adjusted by tuning their chemical composition so that they do not absorb light of the wavelength(s) of the available light source. In various embodiments, the light source could include one or more of a laser, a laser diode, an LED or an IPL source. For reasons of skin safety, the wavelengths of the light are preferably 400 nm or higher.

Selective heating of hair results in the hair being heated up to a higher temperature than that of the containers 100 and the compounds 110 encapsulated therein. Because of the temperature differences between the hair and the containers 100, some of the heat from the hair is transferred to the containers 100 that are in thermal contact with the hair, until the hair and the containers 100 are in thermodynamic equilibrium. Since the temperature of the containers 100 is not increased directly (or onlyat a lower rate) by the heat source providing the selective heating, such selective heating of the hair ensures that the containers 100 may only reach their transition temperature via the heat transfer from the hair.

The thermal contact between the hair and the containers 100 could be established e.g. by the containers 100 being adjacent to, i.e. in contact with, the hair. The heating of the containers 100 could also be established when the containers 100 are not in direct contact with the hair but are in close proximity of the hair, so that heat transfer from the hair to the containers 100 is enabled via e.g. convection. In some applications of the present invention, the latter type of thermal contact is preferably minimized to ensure that only the containers 100 that are in direct contact with the hair are able to receive sufficient heat from the hair to be heated up to their transition temperature and release the compounds 110. This could be achieved by e.g. selectively heating the hair to a sufficiently high temperature but only for a short period of time. In this manner, the sufficiently high temperature of the hair would enable heat transfer to the containers 100 that are in direct contact with the hair, so that these containers release the compounds 110 encapsulated therein, but the heat transfer to the containers 100 that are not in direct contact with the hair would not be sufficient to heat those containers to their transition temperature. Consequently, the containers 100 that are not in direct contact with the hair would remain closed.

When some of the containers 100 distributed over hair reach, via the heat transfer from the hair, their transition temperature (e.g. hyperthermia temperature of 40-45°C), these containers release the chemical compound 110 encapsulated in them. The chemical compound 110 could be e.g. a compound for hair treatment such as hair removal, hair coloring, and/or hair conditioning. Such a compound could be configured to react with the hair tissue in order to degrade, color, or condition the hair tissue, when the compound is in contact with the hair.

In a preferred embodiment, the containers 100 are selected or modified such that their transition temperature is sufficiently far above the body temperature in order to avoid the containers reaching their transition temperature by being in contact with the body and uncontrollably releasing the compound 110. While the transition temperature should be above the body temperature and, particularly, above the skin temperature, it should also be below the maximum temperature reached by the hair exposed to selective heating. The maximum temperature may vary, depending on the particular application of the disclosed method of releasing the chemical compound 110. For example, in a method where the hair is merely heated by light, the maximum temperature could be as low as 35-50°C. However, in applications where the hair is cut by the heat, such as e.g. laser shaving, the temperature of the hair may locally reach the melting and/or evaporation temperature of the hair, which is typically much higher than 50°C.

In an embodiment, the containers 100 could be in the form of temperature-sensitive liposomes capable of triggered-release of a bioactive ingredient (i.e., the chemical compound) from their lumen. These heat-sensitive vessels release encapsulated molecules at the melting phase transition temperature *(T*ₘ*)* of the lipid bilayer. At *T*ₘ*,* structural changes in the lipid membrane occur as it transfers from a gel to the liquid-crystalline phase. Liposomal membranes in the gel (*i.e.* solid-like) phase are less permeable to water and drugs compared to the liquid-crystalline phase. At the melting phase transition temperature, the membrane permeability of the lipid bilayer increases by several orders of magnitude, thereby facilitating the release of the chemical compound previously encapsulated therein.

The *T*ₘ of the liposomes could be adjusted by tuning the chemical composition of the lipid bilayer, as is known in the art. For example, incorporation of *lyso-*phosphatidylcholines (*lyso-*PCs) in the bilayer of liposomes has a pronounced effect on the release properties of the heat-sensitive container. In this manner, low temperature-sensitive liposomes (LTSLs) composed of *lyso*-PC/DPPC/DPPE-PEG2000 could be created that release encapsulated aqueous solutes in a matter of seconds at 39-42°C.

Some examples of the chemical compound 110 that could be encapsulated in the containers 100 include water-soluble components such as enzymes, hormones or other chemicals like e.g. thioglycolate.

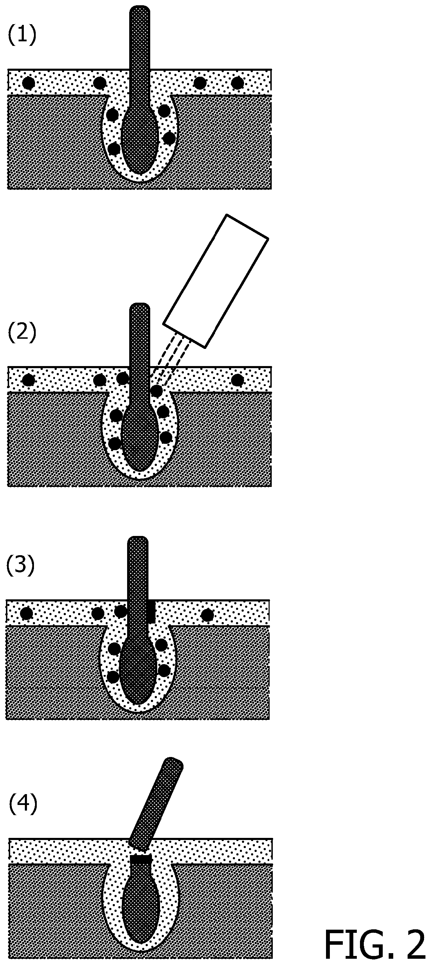

Figs. 2-4 provide some exemplary applications of the method of releasing the chemical compound onto hair, according to various embodiments of the present invention. However, a person skilled in the art will recognize that many further applications are possible, e.g. applications where the released chemical compound does not directly result in treatment of hair, but may serve as a pre-cursor for such treatment later on, all of these applications being within the scope of the present invention.

Fig. 2 illustrates removal of hair by temperature-induced release of bioactive ingredients from temperature-sensitive containers, according to an embodiment of the present invention. As shown in step (1) of Fig. 2, first the containers 100 (indicated by means of the round dots) are distributed over the hair and the skin. In step (2) illustrated in Fig. 2, the hair is locally heated with a light source (shown as a rectangle). The local heating of the hair results in temperature-mediated release of the chemical compound from the containers, illustrated in step (3) of Fig. 2, which reduces the disulfide bridges of keratin in the hair. In step (4) of Fig. 2, the hair is removed and the remainder of the containers and the released chemical compounds could be wiped off. This approach could be used in a depilation product, such as an IPL photoepilation device, or a shaving product.

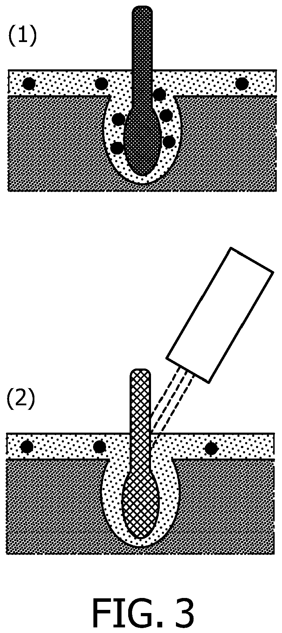

Fig. 3 illustrates release of hair dyes, conditioner or therapeutics from temperature-sensitive containers, according to an embodiment of the present invention. As shown in step (1) of Fig. 3, first the containers 100 (again, shown by means of the round dots) are distributed over the hair and the skin. In step (2) illustrated in Fig. 3, the hair is locally heated with a light source (shown as a rectangle), which results in the release of the dye, conditioner or therapeutic from the containers. The released dye could have a cosmetic function, e.g. when it is used for coloring of the hair, or may serve to enhance the efficacy of a photoepilation treatment.

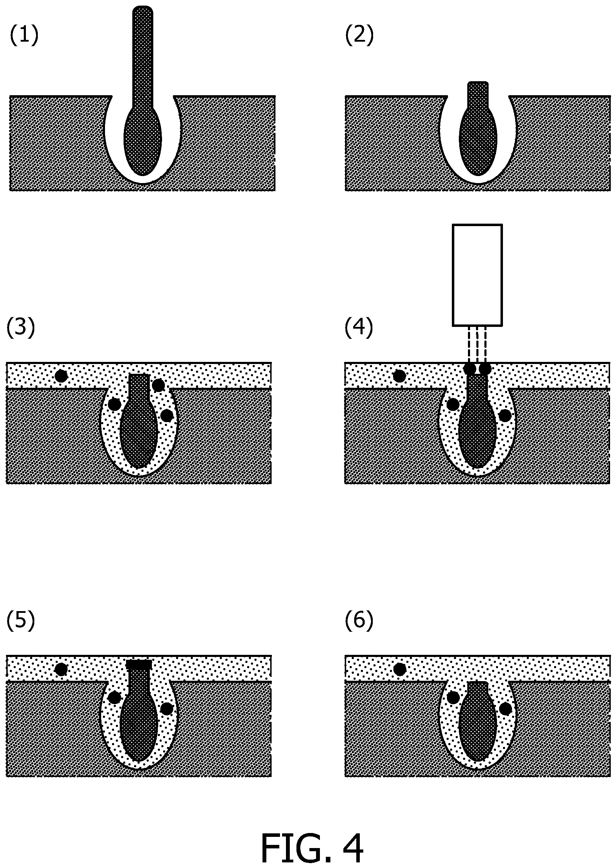

Fig. 4 illustrates enhanced photoepilation, according to an embodiment of the present invention. This embodiment combines photoepilation with temperature-triggered release of bioactive ingredients to the hair. Step (1) of Fig. 4 illustrates the original state of the hair and the skin. Step (2) of Fig. 4 illustrates pre-shaving of the hair, resulting in remaining stubble. Step (3) of Fig. 4 illustrates that the containers 100 (again, shown by means of the round dots) are distributed over the hair and the skin. Step (4) of Fig. 4 illustrates a light source (shown as a rectangle) locally heating up the hair and the hair follicle, thereby locally triggering the release of the hair-attacking chemical compound encapsulated in the containers 100. Step (5) illustrates the released chemical compound dissolving the hair, which results in smoother skin compared to pre-shaving treatment only, as shown in step (6) of Fig. 4.

For the proof of principle, temperature-triggered release of fluorescent molecules from the aqueous lumen of a temperature-sensitive liposome (TSL) in the presence of hair was investigated using a long-pulse commercial photoepilation device and a short-pulse commercial photoepilation device. The long-pulse device was a device characterized by a pulse length between 140 and 360 ms, fluence between 10 and 35 J/cm² , and wavelength of 810 nm. The short-pulse device was a device characterized by a pulse length of 2 ms, fluence between 3 and 6 J/cm², and wavelength between 570 and 1200 nm.

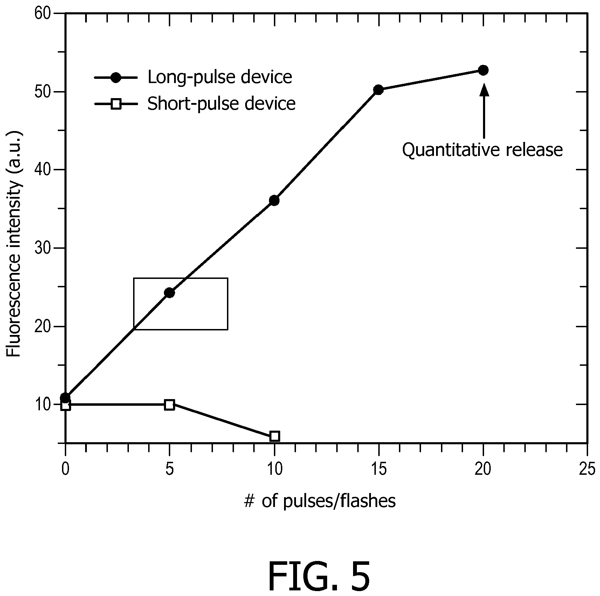

The release of encapsulated molecules from the TSL was probed by fluorescence spectroscopy. As shown in Fig. 5, quantitative release of the fluorescent molecule from the TSL was observed after the application of 20 light pulses using the long-pulse commercial photoepilation device, where pulse duration is of the order of hundreds of milliseconds (ms). As expected, no release of the fluorescent molecule from the TSL was observed after the application of 20 light pulses using the short-pulse photoepilation device, because the pulse duration in that device is only 2 ms. Moreover, control experiments with TSLs and photoepilation devices in the absence of hair didn't show any release of the fluorescent molecules. In conclusion, the release of encapsulated fluorescent probes from TSLs has been demonstrated by heating up the hair by light using a long-pulse photoepilation device.

## Claims

1. A method of releasing a chemical compound, the method comprising:
distributing containers encapsulating the chemical compound over hair, the containers being configured to release the chemical compound upon reaching a transition temperature; and
heating the hair to a predetermined temperature equal to or higher than the transition temperature,
wherein the heating of the hair enables heat transfer from the hair to one or more of the containers that are in proximity of the hair, whereby the one or more containers reach the transition temperature and release the chemical compound.

2. The method according to claim 1, wherein the hair is heated by being irradiated with light, wherein a wavelength or a range of wavelengths of the light is selected so that a portion of the light absorbed by the hair is greater than a portion of the light absorbed by the containers and the chemical compound.

3. The method according to claim 2, wherein the light comprises a light generated by a laser or an intense pulsed light source.

4. The method according to any one of the preceding claims, wherein the containers comprise liposomes.

5. The method according to claim 4, wherein the liposomes comprise *lyso-*PC/DPPC/DPPE-PEG2000.

6. The method according to any one of the preceding claims, wherein the chemical compound comprises one or more of enzymes, hormones, or thioglycolates.

7. The method according to any one of the preceding claims, wherein the chemical compound is configured for treatment and/or removal of the hair that is in contact with the released chemical compound.

8. The method according to any one of the preceding claims, wherein the step of distributing the containers over the hair comprises distributing the containers over a skin area comprising the hair.

## Patentansprüche

1. Verfahren zum Freisetzen einer chemischen Verbindung, Verfahren umfassend:
Verteilen von Behältern, die die chemische Verbindung einkapseln, über Haar, wobei die Behälter konfiguriert sind, um die chemische Verbindung beim Erreichen einer Übergangstemperatur freizusetzen, und
Erwärmen des Haars auf eine vorbestimmte Temperatur gleich oder höher der Übergangstemperatur,
wobei das Erwärmen des Haars Wärmetransfer von dem Haar zu einem oder mehreren der Behälter, die in der Nähe des Haars sind, ermöglicht, wodurch der eine oder die mehreren Behälter die Übergangstemperatur erreicht/erreichen und die chemische Verbindung freisetzen.

2. Verfahren nach Anspruch 1, wobei das Haar erwärmt wird, indem es mit Licht bestrahlt wird, wobei eine Wellenlänge oder ein Bereich von Wellenlängen des Lichts derart ausgewählt ist, dass ein Anteil des Lichts, der von dem Haar absorbiert wird, größer ist als ein Anteil des Lichts, das von den Behältern und der chemischen Verbindung absorbiert wird.

3. Verfahren nach Anspruch 2, wobei das Licht ein Licht umfasst, das von einem Laser oder einer intensiven Quelle gepulsten Lichts erzeugt wird.

4. Verfahren nach einem der vorhergehenden Ansprüche, wobei die Behälter Liposome umfassen.

5. Verfahren nach Anspruch 4, wobei die Liposome *lyso*-PC/DPPC/DPPE-PEG2000 umfassen.

6. Verfahren nach einem der vorhergehenden Ansprüche, wobei die chemische Verbindung Enzyme und/oder Hormone und/oder Thioglycolate umfasst.

7. Verfahren nach einem der vorhergehenden Ansprüche, wobei die chemische Verbindung zur Behandlung und/oder zum Entfernen von Haar, das mit der freigesetzten chemischen Verbindung in Berührung ist, konfiguriert ist.

8. Verfahren nach einem der vorhergehenden Ansprüche, wobei der Schritt des Verteilens der Behälter über das Haar das Verteilen der Behälter über einen Hautbereich, der Haar umfasst, umfasst.

## Revendications

1. Procédé de libération d'un composé chimique, le procédé comprenant :
la répartition de récipients encapsulant le composé chimique sur des cheveux, les récipients étant configurés pour libérer le composé chimique lorsqu'il atteint une température de transition ; et
le chauffage des cheveux jusqu'à une température prédéterminée supérieure ou égale à la température de transition,
dans lequel le chauffage des cheveux permet un transfert de chaleur des cheveux à un ou plusieurs des récipients qui sont à proximité des cheveux, moyennant quoi les un ou plusieurs des récipients atteignent la température de transition et libèrent le composé chimique.

2. Procédé selon la revendication 1, dans lequel les cheveux sont chauffés en étant irradiés de lumière, dans lequel une longueur d'onde ou une plage de longueurs d'onde de la lumière est choisie de sorte qu'une portion de la lumière absorbée par les cheveux soit plus grande qu'une portion de la lumière absorbée par les récipients et le composé chimique.

3. Procédé selon la revendication 2, dans lequel la lumière comprend une lumière générée par un laser ou une source de lumière pulsée intense.

4. Procédé selon l'une quelconque des revendications précédentes, dans lequel les récipients comprennent des liposomes.

5. Procédé selon la revendication 4, dans lequel les liposomes comprennent *lyso*-PC/DPPC/DPPE-PEG2000.

6. Procédé selon l'une quelconque des revendications précédentes, dans lequel le composé chimique comprend un ou plusieurs parmi des enzymes, des hormones, ou des thioglycolates.

7. Procédé selon l'une quelconque des revendications précédentes, dans lequel le composé chimique est configuré pour un traitement et/ou une élimination des cheveux qui sont en contact avec le composé chimique libéré.

8. Procédé selon l'une quelconque des revendications précédentes, dans lequel l'étape de répartition des récipients sur les cheveux comprend la répartition des récipients sur une zone de la peau comprenant les cheveux.
